# NEW EUROPEAN PATENT SPECIFICATION

(11) **EP 1 430 861 B2**
(45) Date of publication and mention of the opposition decision: **03.08.2016**
(45) Mention of the grant of the patent: 14.01.2009
(21) Application number: 02258797.6
(22) Date of filing: 19.12.2002
(51) Int. Cl.: A61F 13/15, D04H 1/00, D04H 3/00

(54) **Perforated plastically deformable sheet and method for making the same**
Perforierte plastisch verformbare Kunststofffolie und zugehöriges Herstellungsverfahren
Feuille perforée plastiquement déformable et son procédé de fabrication

(43) Date of publication of application: 23.06.2004
(73) Proprietor: Huang, Chen-Cheng, Taipey City (TW)
(72) Inventor: Huang, Chen-Cheng, Taipey City (TW)
(74) Representative: MacDougall, Alan John Shaw

(56) References cited:
- EP-A- 0 545 423
- US-A- 4 323 069
- US-A- 4 342 314
- US-A- 4 601 868
- US-A- 5 514 105
- US-A- 5 660 788

## Description

This invention relates to a perforated plastically deformable sheet, more particularly to a perforated plastically deformable sheet with a plurality of recesses and a plurality of capillary units.

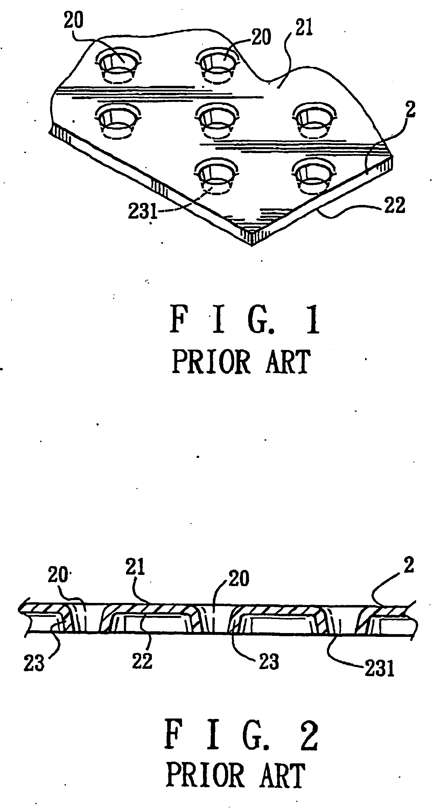

Figs. 1 and 2 illustrate a conventional perforated plastically deformable sheet 2 that can be used as a top sheet of an absorptive device, such as diapers, bandages, water-retaining agent for agriculture use, and sanitary napkins or can be used as packaging and decorating material. The perforated plastically deformable sheet 2 has opposite top and bottom surfaces 21, 22, and is perforated to form a plurality of tapered capillaries 23. Each of the capillaries 23 has a bottom opening 231 spaced apart from the bottom surface 22 in a direction a way from the top surface 21 and adapted to be in intimate contact with an absorbent batt (not shown) so that liquid accumulated on the top surface 21 of the elastically deformable sheet 2 can be absorbed by the absorbent batt through the capillaries 23 (a wicking action). Formation of the capillaries 23 is performed by advancing a softened plastically deformable sheet through a vacuum screen drum, which causes the softened plastically deformable sheet to rupture at screen holes in the vacuum screen drum.

The conventional perforated plastically deformable sheet 2 is disadvantageous in that the height of each of the thus-formed capillaries 23 is relatively small and a sufficient distance between the absorbent batt and the top surface 21 cannot be provided to prevent liquid from wetbacking from the absorbent batt to the top surface 21 (which is in contact with the user's skin). Moreover, since prolonged contact with the perforated plastically deformable sheet 2 can irritate the user's skin, there is a need to minimize the contact area of the perforated plastically deformable sheet 2 with the user's skin.

US Patent 4,342,314 (Procter & Gamble) discloses a resilient plastic web that in two embodiments (Figs. 11 and 12) includes a laminated sheet structure in which each sheet has a respective set of aligned apertures of smaller size from those in an adjacent sheet, the laminated sheet structure thus having tapered apertures for producing a capillary effect.

The object of the present invention is to provide a perforated plastically deformable sheet that is capable of overcoming drawbacks of the prior art.

According to one aspect the present invention provides a perforated plastically deformable sheet as set out in appended claim 1.

In drawings which illustrate embodiments of the invention,
Fig. 1 is a fragmentary perspective view of a conventional perforated plastically-deformable sheet for an absorptive device;
Fig. 2 is fragmentary sectional view of the perforated plastically-deformable sheet of Fig. 1;
Fig. 3 is a fragmentary top view of a preferred embodiment of a perforated plastically-deformable sheet according to this invention;
Fig. 4 is a sectional view taken along line IV-IV in Fig. 3;
Fig. 5 is a schematic side view illustrating how the perforated plastically-deformable sheet of Fig. 3 is made using a vacuum screen drum;
Fig. 6 is a fragmentary perspective view of a screen mold of the vacuum screen drum shown in Fig. 5;
Fig. 7 is a schematic side view of another apparatus for making the perforated plastically deformable sheet according to this invention; and
Fig. 8 is a fragmentary sectional view of a second preferred embodiment of the perforated plastically deformable sheet according to this invention.

For the sake of brevity, like elements are denoted by the same reference numerals throughout the disclosure.

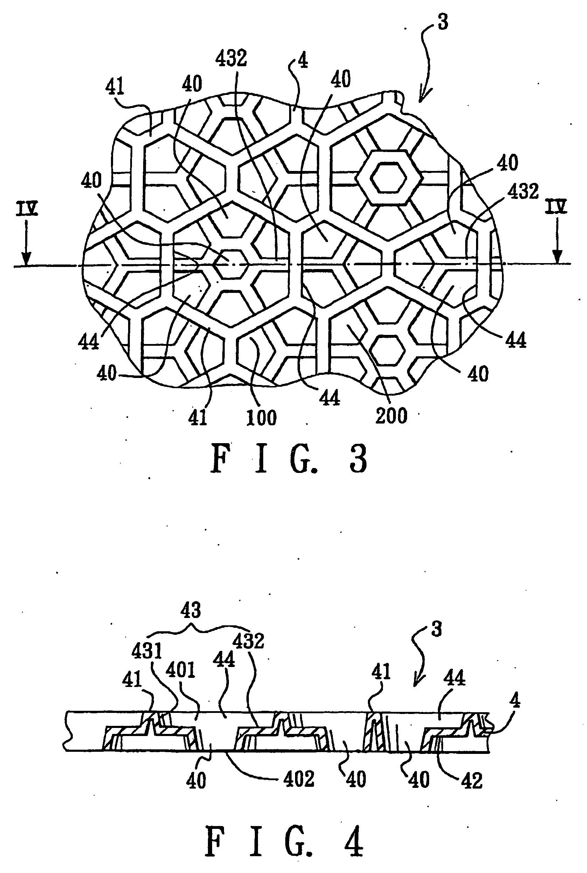

Figs. 3 and 4 illustrate a perforated plastically deformable sheet 3 of this invention that can be used as a top sheet of an absorptive device, such as diapers, bandages, water-retaining agent for agriculture use, and sanitary napkins or can be used as packaging and decorating material.

The perforated plastically deformable sheet 3 includes: a plastically deformable sheet 4 having opposite top and bottom surfaces 41, 42 and formed with a plurality of spaced apart recesses 44 and a plurality of spaced apart capillary units that are vertically and respectively aligned with the recesses 44. Each of the capillary units includes at least one capillary 40 that has a cross-section less than that of the respective one of the recesses 44. Each of the recesses 44 is indented from the top surface 41 toward the 'bottom surface 42, and is confined by a recess-confining wall 43 that has a peripheral surface 431 extending downwardly from the top surface 41 toward the bottom surface 42, and a bight surface 432 that extends laterally from the peripheral surface 431. The capillary 40 of each of the capillary units extends downwardly from the bight surface 432 of the recess-confining wall 43 of the respective one of the recesses 44 in a transverse direction relative to the bight surface 432, and has a top opening 401 adjoining the respective one of the recesses 44 and a bottom opening 402 opposite to the top opening 401 and spaced apart from the bottom surface 42 in a direction away from the top surface 41.

In this embodiment, the recesses 44 are hexagonal in shape, are classified into two different sizes, and have a pattern such that each of the larger ones (indicated as reference numeral 200 in Fig. 3) of the recesses 44 is surrounded by a plurality of the smaller ones (indicated as reference numeral 100 in Fig. 3) of the recesses 44. Each of the larger ones 200 of the recesses 44 spans seven capillaries 40 of a respective one of the capillary units. Each of the smaller ones of the recesses 44 spans three capillaries 40 of a respective one of the capillary units.

The perforated plastically deformable sheet 3 is preferably made from a hydrophobic and non-woven fabric. The non-woven fabric is preferably a fibrous based material which can contain solely a fiber material or a mixture of different fiber materials. Each of thy capillaries 40 of the capillary units is tapered from the top opening 401 toward the bottom opening 402.

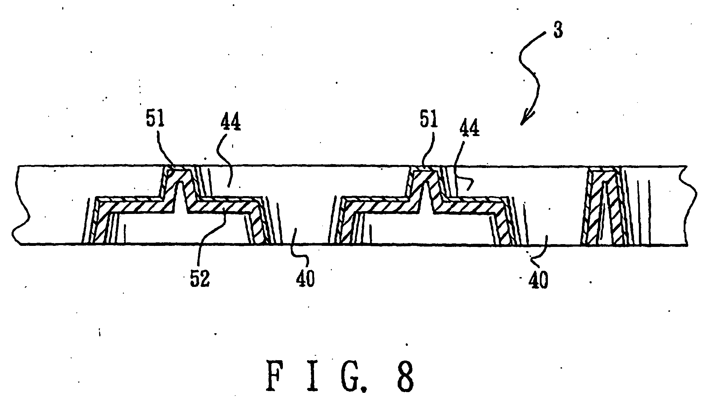

Fig. 8 illustrates a second preferred embodiment of the perforated plastically deformable sheet of this invention that has a structure similar to the previous embodiment shown in Fig. 4, except that the perforated plastically deformable sheet 3 of this embodiment includes a top layer 51 of a hydrophilic material and a bottom layer 52 of a hydrophobic material that is adhered to a bottom side of the top layer 51.

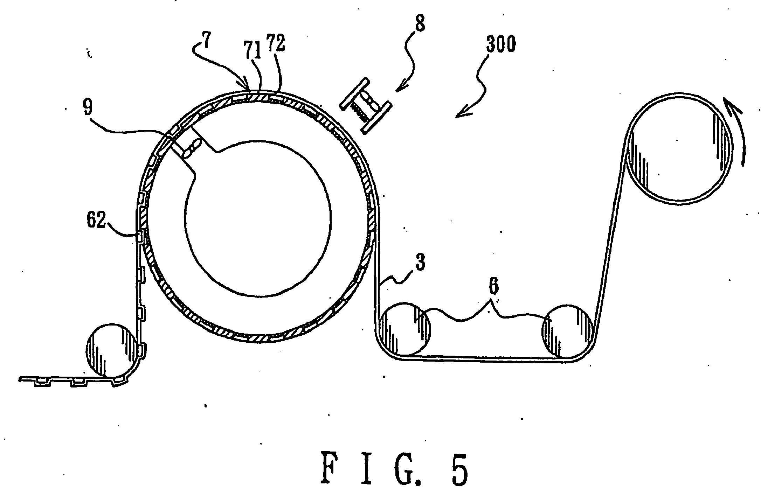

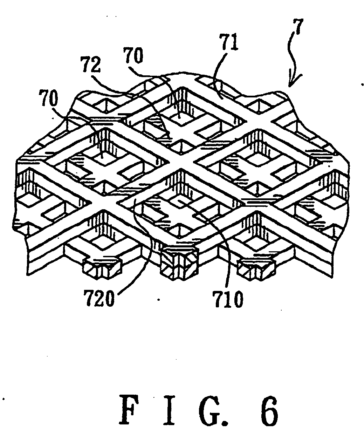

Figs. 5 and 6 illustrate an apparatus 300 for making the perforated plastically deformable sheet 3 according to an exemplary method. The method includes the steps of: (a) preparing a plastically deformable sheet 4; (b) preparing a drum-type screen mold 7 including a first screen element 71 and a second screen element 72 connected to and disposed above the first screen element 71, the first and second screen elements 71, 72 being respectively formed with a plurality of first and second screen holes 710, 720, each of the second screen holes 720 having a cross-section greater than those of the first screen holes 710, each of the second screen holes 720 spanning at least one of the first screen holes 710; (c) heating and softening the plastically deformable sheet 4 using a heater 8, laying the plastically deformable sheet 4 on the second screen element 72, and passing the plastically deformable sheet 4 over the screen mold 7 using a set of rollers 6; and (d) applying a negative pressure at one side of the first screen element 71 opposite to the softened plastically deformable sheet 4 using a vacuum device 9 in such a mannerthatthe softened plastically deformable sheet 4 is pulled and ruptured at the first screen holes 710 so as to form the recesses 44 that respectively correspond to the second screen holes 720 and so as to form the capillaries 40 that respectively correspond to the first screen holes 710.

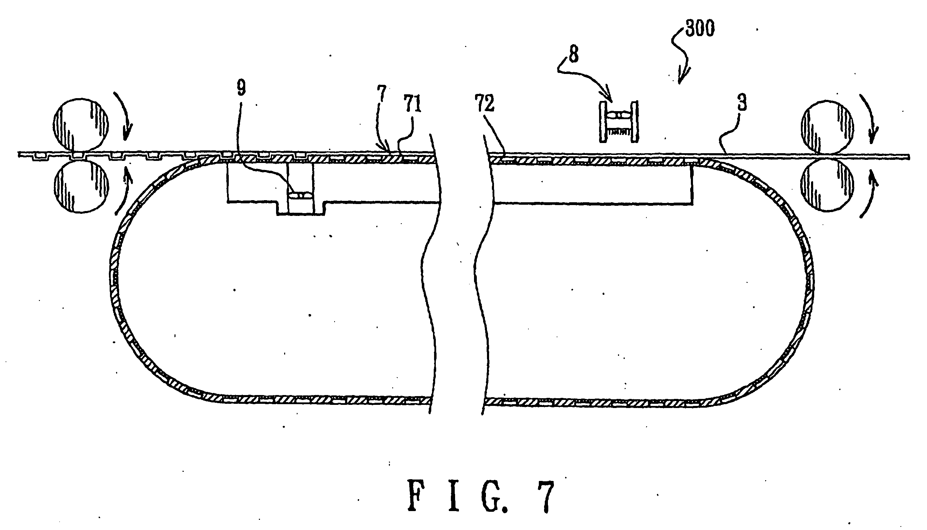

Fig. 7 illustrates another apparatus 300 that is similar to the apparatus 300 shown in Fig. 5, except that the screen mold 7 is elliptical in shape.

With the inclusion of the recesses 44 in the perforated plastically deformable sheet 3, the drawbacks as encountered in the prior art can be eliminated. Moreover, the recesses 44 permit the liquid that accumulated on the top surface 41 of the perforated plastically deformable sheet 3 to be quickly drained thereinto, thereby allowing a greater degree of dryness on the top surface 41 of the perforated plastically deformable sheet 3.

Each feature disclosed in this specification (which term includes the claims) and/or shown in the drawings may be incorporated in the invention independently of other disclosed and/or illustrated features.

Statements in this specification of the "objects of the invention" relate to preferred embodiments of the invention, but not necessarily to all embodiments of the invention falling swithin the claims.

The description of the invention with reference to the drawings is by way of example only.

The text of the abstract filed herewith is repeated here as part of the specification.

A perforated plastically deformable sheet (3) includes a plastically deformable sheet (4) with top and bottom surfaces (41, 42). The plastically deformable sheet (4) is formed with a plurality of recesses (44) and a plurality of capillaries (40), each of which extends from a respective one of the recesses (44). Each of the recesses (44) has a cross-section greater than that of the respective one of the capillaries (40).

## Claims

1. A perforated plastically deformable sheet (3) **characterized by**:
a plastically deformable sheet (4) having opposite top and bottom surfaces (41, 42) and formed with a plurality of spaced apart recesses (44) and a plurality of spaced apart capillary units that are vertically and respectively aligned with said recesses (44), each of said capillary units including at least one capillary (40) that has a cross-section less than that of the respective one of said recesses (44), each of said recesses (44) being indented from said top surface (41) toward said bottom surface (42) and being confined by a recess-confining wall (43) that has a peripheral surface (431) extending downwardly from said top surface (41) toward said bottom surface (42), and a bight surface (432) that extends laterally from said peripheral surface (431), said capillary (40) of each of said capillary units extending downwardly from said bight surface (432) of said recess-confining wall (43) of the respective one of said recesses (40) in a transverse direction relative to said bight surface (432) and having a top opening (401) adjoining the respective one of said recesses (44) and a bottom opening (402) opposite to said top opening (401) and spaced apart from said bottom surface (42) in a direction away from said top surface (41);
wherein said recesses (44) are hexagonal in shape and have two different sizes, each of the larger ones (200) of the recesses (44) is surrounded by a plurality of the smaller ones (100) of the recesses (44), each of the larger recesses (200) spans seven capillaries (40) and each of the smaller recesses (100) spans three capillaries (40), and all of said capillaries (40) are shaped as a polygon that has interior obtuse angles.

2. The perforated plastically deformable sheet (3) of Claim 1, **characterized in that** said capillary (40) of each of said capillary units is tapered from said top opening (401) toward said bottom opening (402).

3. The perforated plastically deformable sheet (3) of Claim 1, **characterized in that** saidplastically deformable sheet (4) is made from a plastic material that is hydrophobic.

4. The perforated plastically deformable sheet (3) of Claim 1, **characterized in that** said plastically deformable sheet (4) includes a top layer (51) of a hydrophilic material and a bottom layer (52) of a hydrophobic material.

5. The perforated plastically deformable sheet (3) of Claim 1, **characterized in that** said plastically deformable sheet (4) is made from a non-woven fabric.

## Patentansprüche

1. Perforierte, plastisch verformbare Folie (3), **gekennzeichnet durch**:
eine plastisch verformbare Folie (4), welche eine obere und eine untere Fläche (41, 42) aufweist, die sich gegenüberliegen, und mit einer Vielzahl beabstandeter Ausnehmungen (44) und einer Vielzahl beabstandeter Kapillareinheiten ausgebildet ist, die vertikal und jeweils an den Ausnehmungen (44) ausgerichtet sind, wobei jede der Kapillareinheiten mindestens eine Kapillare (40) umfasst, deren Querschnitt kleiner ist als der der entsprechenden Ausnehmung (44), wobei jede der Ausnehmungen (44) von der oberen Fläche (41) aus zur unteren Fläche (42) hin vertieft und von einer Ausnehmungsbegrenzungswand (43) begrenzt ist, die eine Umfangsfläche (431), die von der oberen Fläche (41) aus nach unten zu der unteren Fläche (42) hin verläuft, und eine Einbuchtungsfläche (432) aufweist, die von der Umfangsfläche (431) aus seitlich verläuft, wobei die Kapillare (40) jeder Kapillareinheit von der Einbuchtungsfläche (432) der Ausnehmungsbegrenzungswand (43) der entsprechenden Ausnehmung (40) aus in einer in Bezug auf die Einbuchtungsfläche (432) schrägen Richtung nach unten hin verläuft und eine an die jeweilige Ausnehmung (44) angrenzende obere Öffnung (401) und eine der oberen Öffnung (401) gegenüberliegende untere Öffnung (402) aufweist, die in einer von der oberen Fläche (41) wegführenden Richtung von der unteren Fläche (42) beabstandet ist,
wobei die Ausnehmungen (44) eine sechseckige Form und zwei verschiedene Größen aufweisen, jede der größeren (200) Ausnehmungen (44) von einer Vielzahl der kleineren (100) Ausnehmungen (44) umgeben ist, sich jede der größeren Ausnehmungen (200) über sieben Kapillaren (40) und jede der kleineren Ausnehmungen (100) über drei Kapillaren (40) erstreckt und alle Kapillaren (40) wie ein Vieleck geformt sind, das stumpfe Innenwinkel aufweist.

2. Perforierte, plastisch verformbare Folie (3) nach Anspruch 1, **dadurch gekennzeichnet, dass** sich die Kapillare (40) jeder Kapillareinheit von der oberen Öffnung (401) aus zur unteren Öffnung (402) hin verjüngt.

3. Perforierte, plastisch verformbare Folie (3) nach Anspruch 1, **dadurch gekennzeichnet, dass** die plastisch verformbare Folie (4) aus einem Kunststoffmaterial hergestellt ist, das wasserabweisend ist.

4. Perforierte, plastisch verformbare Folie (3) nach Anspruch 1, **dadurch gekennzeichnet, dass** die plastisch verformbare Folie (4) eine obere Schicht (51) aus einem wasseranziehenden Material und eine untere Schicht (52) aus einem wasserabweisenden Material umfasst.

5. Perforierte, plastisch verformbare Folie (3) nach Anspruch 1, **dadurch gekennzeichnet, dass** die plastisch verformbare Folie (4) aus einem Vliesstoff hergestellt ist.

## Revendications

1. Feuille perforée plastiquement déformable (3) **caractérisée par** :
une feuille plastiquement déformable (4) ayant des surfaces supérieure et inférieure opposées (41, 42) et formée avec une pluralité de creux espacés (44) et une pluralité d'unités capillaires espacées qui sont alignées verticalement et respectivement avec lesdits creux (44), chacune desdites unités capillaires comprenant au moins un capillaire (40) ayant une section transversale inférieure à celle du creux respectif desdits creux (44), chacun desdits creux (44) étant dentelé de ladite surface supérieure (41) vers ladite surface inférieure (42), et délimité par une paroi de délimitation de creux (43) qui a une surface périphérique (431) s'étendant vers le bas à partir de ladite surface supérieure (41) vers ladite surface inférieure (42), et une surface de plateau (432) qui s'étend latéralement à partir de ladite surface périphérique (431), ledit capillaire (40) de chacune desdites unités capillaires s'étendant vers le bas à partir de ladite surface de plateau (432) de ladite paroi de délimitation de creux (43) de celui respectif desdits creux (40) dans une direction transversale par rapport à ladite surface de plateau (432) et ayant une ouverture supérieure (401) contiguë à celui respectif desdits creux (44) et une ouverture inférieure (402) opposée à ladite ouverture supérieure (401) et espacée de ladite surface inférieure (42) dans une direction s'éloignant de ladite surface supérieure (41) ;
dans laquelle lesdits creux (44) ont une forme hexagonale et deux tailles différentes, chacun des plus grands (200) des creux (44) étant entouré par une pluralité des plus petits (100) des creux (44), chacun des plus grands creux (200) enjambant sept capillaires (40) et chacun des plus petits creux (100) enjambant trois capillaires (40), et tous lesdits capillaires (40) ayant une forme polygonal avec des angles internes obtus.

2. Feuille perforée plastiquement déformable (3) selon la revendication 1, **caractérisée en ce que** ledit capillaire (40) de chacune desdites unités capillaires est conique de ladite ouverture supérieure (401) vers ladite ouverture inférieure (402).

3. Feuille perforée plastiquement déformable (3) selon la revendication 1, **caractérisée en ce que** ladite feuille plastiquement déformable (4) est réalisée à partir d'un matériau plastique qui est hydrophobe.

4. Feuille perforée plastiquement déformable (3) selon la revendication 1, **caractérisée en ce que** ladite feuille plastiquement déformable (4) comprend une couche supérieure (51) d'un matériau hydrophile et une couche inférieure (52) d'un matériau hydrophobe.

5. Feuille perforée plastiquement déformable (3) selon la revendication 1, **caractérisée en ce que** ladite feuille plastiquement déformable (4) est réalisée à partir d'une toile non-tissé.
